(19) **Europäisches Patentamt**
**European Patent Office**
**Office européen des brevets**

(11) Veröffentlichungsnummer: **0 295 554 B1**

(12) # EUROPÄISCHE PATENTSCHRIFT

(45) Veröffentlichungstag der Patentschrift:
**17.04.91 Patentblatt 91/16**

(51) Int. Cl.$^5$: **C07C 69/67, C07C 67/38**

(21) Anmeldenummer: **88109093.0**

(22) Anmeldetag: **08.06.88**

(54) **Verfahren zur Herstellung von Omega-Formylalkancarbonsäureestern.**

(30) Priorität: **15.06.87 DE 3719938**

(43) Veröffentlichungstag der Anmeldung:
**21.12.88 Patentblatt 88/51**

(45) Bekanntmachung des Hinweises auf die
Patenterteilung:
**17.04.91 Patentblatt 91/16**

(84) Benannte Vertragsstaaten:
**BE CH DE ES FR GB IT LI NL**

(56) Entgegenhaltungen:
**EP-A- 0 125 567**
**EP-A- 0 173 226**
**DE-A- 2 643 205**
**US-A- 3 253 018**

(73) Patentinhaber: **BASF Aktiengesellschaft**
**Carl-Bosch-Strasse 38**
**W-6700 Ludwigshafen (DE)**

(72) Erfinder: **Bertleff, Werner, Dr.**
**Neuzenlache 3**
**W-6806 Viernheim (DE)**
Erfinder: **Maerkl, Robert, Dr.**
**Bolander Weg 23**
**W-6701 Fussgoenheim (DE)**
Erfinder: **Roeper, Michael, Dr.**
**Albert-Schweitzer-Strasse 10**
**W-6706 Wachenheim (DE)**

## Beschreibung

ω-Formylalkancarbonsäureester sind gesuchte Ausgangsstoffe zur Herstellung von ω-Aminocarbonsäuren, Monomeren zur Herstellung von Polyamiden. Aus der europäischen Patentanmeldung 125 567 ist bekannt, daß man 3-Pentensäureester zunächst zu 4-Pentensäureester isomerisiert und anschließend hydroformyliert und hydriert, um 6-Aminocapronsäureester zu erhalten. Die Isomerisierung ist jedoch ein technisch aufwendiger Schritt.

Die Hydroformylierung von α,β-ungesättigten Alkencarbonsäureestern in Gegenwart von Kolbaltkatalysatoren wird auch in C.R. Acad. Sci. Ser. C 272 (1971) 1, Seiten 86-88, beschrieben. Dort wird 3-Methylbut-2-en-säuremethylester mit Kobaltoctacarbonyl als Katalysator bei 140°C und 180 bar mit einem Gemisch aus Kohlenmonoxid und Wasserstoff umgesetzt. Man erhält 3-Methyl-4-formylbuttersäureester in einer Ausbeute von 72%. Der Rest ist das Hydrierprodukt Isovaleriansäuremethylester. Die Hydrierung als Nebenreaktion wird dort als unvermeidlich angegeben.

Desweiteren wird die Hydroformylierung von 2-Hexensäuremethylester in Gegenwart von Kolbatoctacarbonyl in Chemiker-Zeitung 1976, Heft 7/8, Seite 309, beschrieben. Die Umsetzung wird in starker Verdünnung mit Benzol bei 90°C und 140 bar durchgeführt. Hierbei wird lediglich ein Linearanteil an Formylhexansäuremethylester von 53% erzielt. Darüber hinaus ist es aufwendig, große Mengen Lösungsmittel anzuwenden.

In der US-Patentschrift 3 253 018 wird die Hydroformylierung von Gemischen aus isomeren Pentensäureestern zu Formylvaleriansäureestern in Gegenwart von Kobaltkatalysatoren beschrieben. Dort wird 2-Pentensäureester als wenig geeignet zur Herstellung von 5-Formylvaleriansäureestern bezeichnet, da er am langsamsten reagiert und zur Polymerisation neigt. α,β-ungesättigte Alkencarbonsäureester sind jedoch aufgrund konjugativer Effekte jeweils die stabilsten und durch Isomerisierung am leichtesten erhältlich. Ihre Verwendung zur Herstellung von ω-Formylalkancarbonsäureestern erschien nicht angezeigt.

Es war deshalb die technische Aufgabe gestellt, ausgehend von α,β-ungesättigten Alkencarbonsäureestern ohne vorherige Isomerisierung durch Hydroformylierung ω-Formylalkancarbonsäureester herzustellen und hierbei den Anfall an Nebenprodukten zu vermindern.

Diese Aufgabe wird gelöst in einem Verfahren zur Herstellung von ω-Formylalkancarbonsäureestern durch Hydroformylieren von Alkencarbonsäureestern mit Kohlenmonoxid und Wasserstoff bei erhöhter Temperatur und unter erhöhtem Druck in Gegenwart von Kobaltcarbonylkomplexen, dadurch gekennzeichnet, daß man α,β-ungesättigte Alkencarbonsäureester in Gegenwart von 0,01 bis 1 Mol% Kolbatcarbonylkomplexen, berechnet als Kobalt, unter Einhaltung eines Umsatzes an α,β-ungesättigten Alkencarbonsäureestern von 10 bis 70% hydroformyliert.

Das neue Verfahren hat den Vorteil, daß man vor der Hydroformylierung den aufwendigen Schritt zur Isomerisierung zu endständig ungesättigten Alkencarbonsäureestern vermeidet und von α,β-ungesättigten Alkensäureestern ausgeht. Ferner hat das neue Verfahren den Vorteil, daß die Bildung von Nebenprodukten vermindert wird.

Als Ausgangsverbindungen werden erfindungsgemäß α,β-ungesättigte Alkencarbonsäureester verwendet. Im allgemeinen geht man von α,β-ungesättigten $C_4$-$C_6$-Alkensäureestern aus, die sich vorzugsweise von Alkanolen mit 1 bis 12 Kohlenstoffatomen oder Cycloalkanolen mit 5 bis 8 Kohlenstoffatomen ableiten. Vorzugsweise werden geradkettige α,β-ungesättigte $C_4$-$C_6$-Alkensäureester von Alkanolen mit 1 bis 4 Kohlenstoffatomen als Ausgangsstoffe verwendet. Geeignete Ausgangsverbindungen sind beispielsweise Methyl-, Ethyl-, Propyl-, Butyl-, Hexyl-, Octyl-, Cyclopentyl-, Cyclohexylester der Crotonsäure, 2-Pentensäure oder 2-Hexensäure. Besondere technische Bedeutung haben 2-Pentensäureester als Ausgangsstoffe erlangt.

Die Hydroformylierung wird bei erhöhter Temperatur, vorteilhaft bei einer Temperatur von 60 bis 130°C, insbesondere von 80 bis 110°C, durchgeführt. Hierbei hält man erhöhten Druck, vorteilhaft von 50 bis 200 bar, insbesondere von 30 bis 180 bar, ein. In der Regel enthält das Gasgemisch Kohlenmonoxid und Wasserstoff im Molverhältnis von 1 : 0,5 bis 1 : 10, insbesondere von 1 : 1 bis 1 : 2.

Die Hydroformylierung wird in Gegenwart von Kobaltcarbonylkomplexen durchgeführt. Vorteilhaft verwendet man Kobaltoctacarbonyl das unter Reaktionsbedingungen in die katalytisch aktive Verbindung übergeht. Andererseits ist es auch vorteilhaft die katalytisch aktiven Kobaltcarbonylkomplexe in situ aus Kobaltsaltzen von Fettsäuren, z.B. deren Acetat, Formiat, Propionat oder Butyrat zu erzeugen. Geeignete Ausgangsverbindungen sind auch Kobaltoxide oder Kobalt in metallischer Form für die Herstellung von geeigneten Katalysatoren. Eine empfehlenswerte Form ist es auch, Kobaltcarbonylwasserstoff in wäßriger Lösung durch Behandeln von Kobaltsalzlösungen mit einem Gemisch aus Kohlenmonoxid und Wasserstoff herzustellen und Kobaltcarbonylwasserstoff anschließend vorteilhaft mit den als Ausgangsstoffen verwendeten Alkencarbonsäureestern zu extrahieren.

Die Hydroformylierung wird in Gegenwart von 0,01 bis 1 Mol%, vorzugsweise 0,05 bis 0,3 Mol%, insbesondere 0,08 bis 0,25 Mol%, Kobaltcarbonylkomplexen, berechnet als Kobalt und bezogen auf eingesetzte α,β-ungesättigte Alkencarbonsäureester durchgeführt. Ferner ist es erfindungswesentlich, daß man einen Umsatz von 10 bis 70%, vorzugsweise 15 bis 50%, insbesondere 20 bis 40% an α,β-ungesättigten Alkencarbonsäureestern einhält. Hierdurch wird eine Selektivität von etwa 70% an ω-Formylcarbonsäureestern erzielt und lediglich 4 bis 5% entfallen auf Hydrierprodukte und hochsiedende Komponenten.

Das Hydroformylierungsgemisch wird nach dem Entspannen nach bekannten Methoden aufgearbeitet. Eine geeignete Methode wird z.B. beschrieben in der europäischen Patentschrift 31 100. Hierbei wird der Hydroformylierungsaustrag nach dem Entspannen mit Oxidationsmitteln wie Wasserstoffperoxid oder molekularen Sauerstoff enthaltenden Gasen, insbesondere Luft, vorteilhaft in einer Menge von 2 bis 10 Oxidationsäquivalenten je Mol Kobaltkatalysator in Gegenwart einer wäßrigen sauren Lösung, z.B. wäßrige Ameisensäure oder Essigsäure, Buttersäure, Valeriansäure oder 2-Ethylhexansäure bei einer Temperatur z.B. von 80 bis 160°C, insbesondere 100 bis 130°C, behandelt. Je nach dem Grad der Durchmischung ist die Abtrennung des Kobaltkatalysators bereits nach wenigen Sekunden oder Bruchteilen von Sekunden beendet. Die Kobalt enthaltende wäßrige Phase wird zweckmäßig durch Dekantieren abgetrennt und wiederum zur Herstellung des Katalysators verwendet. Aus der anfallenden organischen Phase werden durch Destillation ω-Formylalkancarbonsäureester isoliert und nicht umgesetzte α,β-ungesättigte Alkencarbonsäureester wiederum der Hydroformylierung zugeführt.

Das Verfahren nach der Erfindung sei an folgenden Beispielen veranschaulicht. Es bedeuten PSE : Pentensäuremethylester, FVSE : Formylvaleriansäuremethylester, HSE : Hexensäuremethylester, FHSE : Formylhexansäuremethylester.

## Beispiele 1 (Vergleich) und 2

Die Versuche wurden in einer kontinuierlich arbeitenden Apparatur durchgeführt. Diese besaß eine Flüssigdosierpumpe, mit der Pentensäuremethylester und der gelöste Kobaltkatalysator in die 2 hintereinander geschalteten Rührautoklaven gefördert wurden. Das Synthesegas wurde druckgeregelt mit dem Flüssigzulauf vor dem ersten Reaktor zusammengeführt. Die beiden Reaktoren besaßen Flüssigvolumina von 1,2 bzw. 1,12 l. Der zweiphasige Austrag wurde unter Druck in einem Behälter gesammelt, aus dem eine bestimmte Abgasmenge abgeregelt wurde. Die Flüssigphase wurde standgeregelt in ein Vorlagegefäß entspannt.

Als Katalysatorvorstufe wurde $CO_2(CO)_8$ eingesetzt und die Hydroformylierung mit $CO/H_2$ = 1 durchgeführt. Für Beispiel 1 wurde 3-Pentensäuremethylester, für Beispiel 2 2-trans-Pentensäuremethylester eingesetzt. Die Reaktion wurde bei 100°C und 90 bar mit einer Belastung von 0,15 l × 1⁻¹ × h⁻¹ durchgeführt.

| Bsp. | $\dfrac{Mol\ Co}{Mol\ PSE}$ × 100[%] | % Umsatz | % n-Anteil[1] | % Sel. 5-FVSE |
|---|---|---|---|---|
| 1 (Vergleich) | 0,16 | 32,8 | 68,7 | 67,9 |
| 2 | 0,16 | 49,9 | 72,2 | 69,2 |

1) Anteil an 5-Formylvalerianester (5-FVSE) am gesamten Formylvaleriansäureester

## Beispiele 3 (Vergleich) und 4

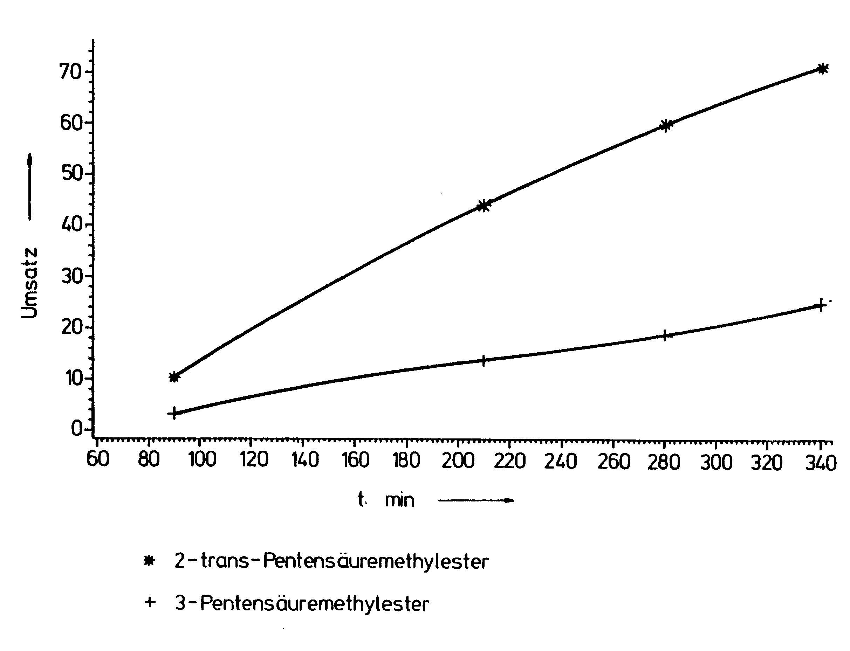

Diese Versuche wurden in einem 2,5 l Hubrührautoklaven durchgeführt. Es wurden jeweils 340 g 3-Pentensäuremethylester (Bsp. 3) bzw. 340 g 2-trans-Pentensäuremethylester (Bsp. 4) und 0,79 g $CO_2(CO)_8$ (0,16 Mol.-% Kobalt) vorgelegt. Die Reaktion wurde bei 75 bis 80 bar $CO/H_2$ = 1 und 100°C durchgeführt. Der Fortgang der Reaktion wurde durch Probenahme nach 90, 210, 280 und 340 Minuten bestimmt. Aus der beigefügten Figur ist ersichtlich, daß 2-trans-Pentensäuremethylester schneller hydroformyliert wird im Vergleich zu 3-Pentensäuremethylester.

## Beispiele 5 (Vergleich) und 6

Diese Versuche wurden analog den Beispielen 3 und 4 durchgeführt, jedoch mit Hexensäuremethylester.

Es wurden jeweils 340 g des Esters eingesetzt. Für Beispiel 5 wurde ein Gemisch, wie es aus der Spaltung von Caprolacton resultiert, mit 66% 5-, 20% 4-trans und 12,6% 4-cis-Isomer eingesetzt, für Beispiel 6 wurde 2-trans-Hexensäuremethylester eingesetzt. Die Kobaltkonzentration betrug 0,18 Mol.-% und die Reaktionsbedingungen waren 75 bis 80 bar $CO/H_2 = 1$, 100°C, 90 min.

| Bsp. | Mol Co ------ x 100[%] Mol HSE | % Umsatz | % n-Anteil[1] | % Sel. 6-FHSE[2] |
|---|---|---|---|---|
| 5 (Vergleich) | 0,18 | 17,6 | 65,9 | 65,8 |
| 6 | 0,18 | 16 | 72,2 | 67,3 |

1) Anteil an 6-Formylhexansäuremethylester am gesamten Formylhexansäureester

2) FHSE = Formylhexansäuremethylester

## Ansprüche

1. Verfahren zur Herstellung von Ω-Formylalkancarbonsäureestern durch Hydroformylieren von isomerisierbaren Alkencarbonsäureestern mit Kohlenmonoxid und Wasserstoff bei erhöhter Temperatur und unter erhöhtem Druck in Gegenwart von Kobaltcarbonylkomplexen, dadurch gekennzeichnet, daß man α,β-ungesättigte isomerisierbare Alkencarbonsäureester in Gegenwart von 0,01 bis 1 Mol-% Kobaltcarbonylkomplexen, berechnet als Kobalt, unter Einhaltung eines Umsatzes an α,β-ungesättigten isomerisierbaren Alkencarbonsäureestern von 10 bis 70% hydroformyliert.

2. Verfahren nach Anspruch 1, dadurch gekennzeichnet, daß man 0,05 bis 0,3 Mol% Kobaltcarbonylkomplexe, berechnet als Kobalt, anwendet.

3. Verfahren nach den Ansprüchen 1 bis 2, dadurch gekennzeichnet, daß man einen Umsatz von 15 bis 50% einhält.

4. Verfahren nach den Ansprüchen 1 bis 3, dadurch gekennzeichnet, daß man eine Temperatur von 60 bis 130°C einhält.

5. Verfahren nach den Ansprüchen 1 bis 4, dadurch gekennzeichnet, daß man einen Druck von 50 bis 200 bar einhält.

6. Verfahren nach den Ansprüchen 1 bis 5, dadurch gekennzeichnet, daß man α,β-ungesättigte $C_4$-$C_6$-Alkensäurealkylester verwendet.

7. Verfahren nach den Ansprüchen 1 bis 6, dadurch gekennzeichnet, daß man 2-Pentensäure-$C_1$-$C_4$-Alkylester verwendet.

## Claims

1. A process for preparing an Ω-formylalkanecarboxylicester by hydroformylating an isomerizable alkenecarboxylic ester with carbon monoxide and hydrogen at elevated temperature and pressure in the presence of a cobalt carbonyl complex, which comprises hydroformylating an α,β-unsaturated isomerizable alkenecarboxylic ester in the presence of from 0.01 to 1 mol% of a cobalt carbonyl complex, calculated as cobalt, while maintaining a conversion rate in respect of the α,β-unsaturated isomerizable alkenecarboxylic ester of from 10 to 70%.

2. A process as claimed in claim 1, wherein from 0.05 to 0.3 mol%, calculated as cobalt, of a cobalt carbonyl complex is used.

3. A process as claimed in claim 1 or 2, wherein a conversion of from 15 to 50% is maintained.

4. A process as claimed in any of claims 1 to 3, wherein a temperature of from 60 to 130°C is maintained.

5. A process as claimed in any of claims 1 to 4, wherein a pressure of from 50 to 200 bar is maintained.

6. A process as claimed in any of claims 1 to 5, wherein an α,β-unsaturated alkyl $C_4$-$C_6$-alkenoate is used.

7. A process as claimed in any of claims 1 to 6, wherein a $C_1$-$C_4$-alkyl 2-pentenoate is used.

**Revendications**

1. Procédé de préparation d'esters d'acides ω-formylalcanecarboxyliques par hydroformylation d'esters d'acides alcènecarboxyliques isomérisables avec du monoxyde de carbone et de l'hydrogène, à température élevée et sous pression élevée, en présence de complexes cobalt-carbonyle, caractérisé en ce qu'on hydroformyle des esters d'acides alcènecarboxyliques isomérisables α,β-insaturés, en présence de 0,01 à 1% en moles de complexes cobalt-carbonyle, calculés en tant que cobalt, en observant un degré de transformation de 10 à 70% des esters d'acides alcènecarboxyliques isomérisables α,β-insaturés.

2. Procédé selon la revendication 1, caractérisé en ce qu'on utilise de 0,05 à 0,3% en moles de complexes cobalt-carbonyle, calculés en tant que cobalt.

3. Procédé selon la revendication 1 ou 2, caractérisé en ce qu'on observe un degré de transformation de 15 à 50%.

4. Procédé selon l'une quelconque des revendications 1 à 3, caractérisé en ce qu'on observe une température de 60 à 130°C.

5. Procédé selon l'une quelconque des revendications 1 à 4, caractérisé en ce qu'on observe une pression de 50 à 200 bars.

6. Procédé selon l'une quelconque des revendications 1 à 5, caractérisé en ce qu'on utilise des esters allyliques d'acides alcénecarboxyliques en $C_4$-$C_6$ α,β-insaturés.

7. Procédé selon l'une quelconque des revendications 1 à 6, caractérisé en ce qu'on utilise des esters alkyliques en $C_1$-$C_4$ d'acide 2-penténique.